# EUROPEAN PATENT APPLICATION

(11) **EP 1 964 562 A1**
(43) Date of publication of application: **03.09.2008**
(21) Application number: 06783809.4
(22) Date of filing: 27.07.2006
(51) Int. Cl.: A61K 31/426, A61K 31/155, A61P 3/10

(54) **PHARMACEUTICAL COMPOSITIONS CONTAINING DERIVATIVE SUBSTANCES OF THIAZOLIDINEDIONES COMBINED WITH A BIGUANIDE FOR USE IN TYPE 2 DIABETES MELLITUS**

(30) Priority: 20.12.2005 MX PA05014092
(71) Applicant: Espinosa Abdala, Leopoldo de Jesús, C.P. 45110 Zapopan, Jalisco , México (MX); WORLD-TRADE IMPORT-EXPORT, WTIE, A.G., 6302 Zug (CH)
(72) Inventor: GARCÍA ARMENTA, María Elena, C.P. 45020 Guadalajara, Jalisco (MX); ALVAREZ OCHOA, Victor Guillermo, C.P. 45222 Zapopan, Jalisco, México (MX); SANTOS MURILLO, Josefina, C.P. 44600 Zapopan, Jalisco (MX); GARCIA ARMENTA, Patricia, C.P. 45110 Zapopan, Jalisco, México (MX)
(74) Representative: Carvajal y Urquijo, Isabel
(86) International application number: PCT/MX2006/000080
(87) International publication number: WO 2007/073136

(57) **Abstract**

The present invention relates to the pharmaceutical industry in general and in particular to the pharmaceutical industry involved in the production of medicaments for controlling type 2 diabetes mellitus. The invention is advantageous over compositions from the prior art in that less of the components are used than when they are consumed separately such as to prevent side or collateral effects, but the same performance or a better performance is achieved nonetheless with said smaller quantities. The inventive composition comprises a pharmaceutical composition containing a substance from the thiazolidinedione group and a biguanide. The composition is **characterized in that** the aforementioned combination or association consists of a selected substance from the thiazolidinedione group, known as Pioglitazone, and a biguanide known as Metformin, in order to produce a product which has a synergistic effect for the treatment and control of type 2 diabetes mellitus, with fewer side effects.

## Description

### FIELD OF THE INVENTION

The present invention generally relates to pharmaceutical industry and, in particular, to drug preparer pharmaceutical industry for controlling type-2 Diabetes Mellitus. More specifically, the invention relates to a composition containing a combination of derivatives of thiazolidinediones (glitazones) such as Pioglitazone and a biguanide such as Metformin, for pharmaceutical use of the composition in type-2 Diabetes Mellitus.

### BACKGROUND OF THE INVENTION

Diabetes represents one of the main public health problems in Mexico. Mexico ranks among the countries with the largest number of registered cases worldwide. The future perspective indicates that the number of people with diabetes will continue to increase. According to the available information, Mexico ranked tenth worldwide in 1995, with 4 million of people with diabetes, and it is estimated that by 2025, it will rank seventh with over 12 million. This statement is confirmed by observing the following data: 40 thousand deaths due to diabetes are yearly registered in Mexico.

The mortality rate per 100 thousand inhabitants in 1981 was 21.4 and increased to 33.4 in 1993, but reached to 43.5 in 1998, the year when 336 thousand 967 cases were reported, and thus to each registered death corresponded about 8 cases. From the above-mentioned, it can be affirm that 38 new cases of diabetes are diagnosed every hour in Mexico.

Type-2 diabetes mellitus (DM2) is a growing importance disease, in both developed and developing countries, whether its importance is measured through the incidence, prevalence or mortality thereof. In the last years, the incidence of DM2 has gradually increased. Although this increase is attributable to "aging" of the population to a certain extent, since it has been observed that incidence increases as age increases, it can be affirmed that the occurrence thereof is on the increase.

In Mexico, DM2 ranked for the first time among the 10 leading causes of death in 1978, and for the group of 55 to 64 years of age, it is the first cause of death. The disease incidence is still unknown and as for prevalence, there are only partial and non-recent studies. However, in people older than 35 years, DM2 ranks as one of the 10 first places of death in all age-groups.

In a study on mortality due to DM2 by the Mexican Institute for Social Security (IMSS), this disease was considered as the main cause of death in Mexico and the state of Nuevo Leon to the Northeast of the country presented the highest age-adjusted mortality rate in all the country.

DM2 is a multifactorial disease. This means that the phenotype results from the interaction of the accumulated effect from various genes, with the effect of important influences from the environment where the individual develops. In diseases which result from multifactorial heritage, it is not possible to infer the genetic constitution of the people, as it is unknown how many and which the participating genes are and if these are autosomal or sex-related genes. What is transmitted from parents to children is a predisposition to develop certain condition, which is to be manifested in case a propitious environment is added.

Within the risk factors for the development of DM2, environmental factors related to lifestyle have been considered. Societies with an occidental lifestyle or a rapid modernization process have shown higher incidence rates. As an example, the case of Japanese immigrants can be cited with higher rates that those living in Japan. Other risk factors could be the diet, obesity, physical inactivity o else the ethnic or genetic structures of the population.

The risk for the development of type-2 diabetes gradually increases to the extent that body weight is increased. In order for a person to develop type-2 diabetes, two types of defects must be present: insulin-resistance and abnormal insulin secretion. In an individual gaining too much weigh, the fat inside abdomen begins to release free fatty acids and certain hormones and substances (TNF-alpha, IL-6, leptin, etc.) which make his own insulin not to work properly. At the beginning, blood-sugar levels are not altered by this insulin-resistance because pancreatic beta-cells produce more insulin. However, a time arrives when the beta-cells become «exhausted», «tired», stop producing the required amount of insulin, and then diabetes occurs.

Both defects have a genetic as well as an environmental basis. As such, a person can be born with «genes» of insulin-resistance, but this resistance will only be expressed and become magnified when the individual gains weight. The maximum insulin secretion response by the beta-cells to an insulin-resistance state may also be genetically predetermined, but a moderated blood-sugar increase and high levels of blood free fatty acids may affect this maximum response and reduce insulin secretion (glucotoxicity and lipotoxicity). Moreover, if there is an intrauterine malnutrition during fetal life, the beta-cells will be badly programmed and, in the future, as an adult, they will not properly release insulin as they will easily become exhausted.

The unexpected increase of type-2 diabetes, particularly in developing countries, among ethnic minorities and very young children and people, seems to be mainly related to the increase in percentage of population with overweight and obesity. However, not all people with overweight or obesity develops diabetes and not all people with type-2 diabetes are obese. The likelihood of developing diabetes when suffering from overweight or obesity depends on the interaction of a series of factors: intra-abdominal fat amount, genetic predisposition to develop insulin-resistance and capacity of insulin production of the person.

There are certain characteristics whereby a person with overweight or obesity increases the risk to develop type-2 diabetes. These are: obesity during childhood and adolescence, progressive weight gain from the age of 18 years, abdominal obesity. Passivity (the absence of physical activity and exercise), high-fat and low-fiber diet, part of an ethnical group with high diabetes prevalence.

The ability of insulin to induce its biological effects over glucose metabolism is decreased in various circumstances. Such is the case of obesity, aging, endocrine disorders due to excessive contra-regulation hormone (glucagon), some genetic alterations and particularly type-2 diabetes.

Up to date, various mechanisms have been postulated whereby insulin-resistance is developed, which comprise pre-receptor defects (because an abnormal insulin molecule is produced or by the presence of antibodies against insulin), receptor defects (as a result of specific mutations) or post-receptor defects, which involve both mutations of glucose transport molecules and low synthesis of transporters and alterations of GLUT-4 translocation.

The mutations of the genes that codify for the different glucose transporters are rare and among them various alterations of proteins GLUT-1, GLUT-2 y GLUT-4 has been identified. In patients with genetic charts of insulin-resistance (due to molecular defects in the insulin receptor), it is possible to observe important growth disorders, adipose tissue atrophy, acanthosis nigricans and, in women, hyperandrogenism with ovarian dysfunction. However, affected individuals do not develop diabetes mellitus unless they also have a genetic susceptibility to secretory dysfunction of pancreatic beta-cell.

In other instances, quite usual, insulin-resistance appears because of a disorder of autoimmune origin, related to the presence of blocking antibodies of hormonal action. As to type-2 diabetes mellitus, insulin-resistance appears to depend neither on anomalies in insulin receptor nor on mechanisms preventing hormone-receptor interaction, but in most of cases it is determined by the presence of post-receptor alterations.

While mutations in glucose transport proteins (particularly GLUT-4) may cause insulin-resistance, such alterations are very rare and the performed studies in humans show that the prevalence thereof is the same in healthy people and in patients with type-2 diabetes mellitus.

Thanks to the researches conducted in the last years, it has been possible to establish that the main defect determining the occurrence of insulin-resistance relates to disorders in translocation of glucose transport molecules and the phosphorylation cascade induced by the interaction between insulin and its receptor. Moreover, as phosphorylation of enzyme phosphoinositolkinase 3 and kinases B and C is fundamental for migration of intracellular vesicles containing GLUT 4 to the membrane, it is evident that the anomalies above mentioned are closely related.

The studies performed in obese individuals and in patients with diabetes mellitus have found a decreased activation of the enzyme phosphoinositolkinase 3, as well as the increase of a substrate molecule of protein kinase C. In this respect, it is worth mentioning that the increase of the expression of the latter in cell cultures associates to a decreased insulin-induced GLUT-4 translocation and, therefore, the glucose transport decreases.

Insulin-resistance specially manifests in peripheral tissue such as muscles and adipose tissue, due to a low retention rate and oxidation of glucose molecules. As already mentioned, compensating hyperinsulinemia is precisely the mechanism whereby an insulin-resistant individual is able to maintain a normal tolerance to carbohydrates. When said mechanism is insufficient due to the appearance of defects in hormonal secretion by pancreatic beta-cells, carbohydrates intolerance occurs and consequently type-2 diabetes.

### Disorders in beta-cell secretion

Several researchers have shown that insulin-resistant individuals, and developing type-2 diabetes mellitus, invariably present a hormone secretion defect, which preferably affects the first stage of this process. Such anomaly, in some instances, can be detected before the appearance of frank hyperglycemia.

Insulin early response, which is how beta-cell capacity to immediately respond to a glucose load has been called, is significantly correlated the hormone with plasma concentration, 30 minutes after the intravenous injection of a standard glucose load.

An important aspect to be considered is the fact that not all insulin-resistant individuals develop type-2 diabetes mellitus, which indicates that the beta-cell defect is essential for the disease to appear clinically manifested. Now then, recent studies show that this defect is present even in individuals with poor glucose tolerance, who have been detected a decrease in the first insulin secretion stage after glucose is orally administered, as well as a lesser capacity of beta-cells to compensate peripheral insulin-resistance and a lesser responsiveness of such cells to blood glucose concentrations.

The beta-cell dysfunction justifies the usage of medicaments stimulating said cell to secrete more insulin in individuals with type-2 diabetes mellitus, whereas peripheral tissue resistance to insulin action indicates that the usage of drugs such as metformin are able to increase hormone-responsiveness.

### Disorders related to insulin-resistance

Insulin-action resistant individuals present a decreased activity of enzyme lipoproteinlipase associated to vascular endothelium; this disorder correlates to the presence of high serum concentrations of triglyceride-rich lipoproteins, particularly those with very low density and low HDL concentration. Another characteristic disorder of plasmatic lipids is the formation of particles smaller and denser than normal that have a higher atherogenic capacity because they are more susceptible to oxidation, due to a low content of antioxidant compounds.

The multiple anomalies associated to poor insulin responsiveness have received, altogether, the appellative of syndrome X. The affected individuals, besides being obese and having hyperinsulinemia at fasting, exhibit the lipid disorders above mentioned and show variables degrees of arterial hypertension, high PIAP-1 concentration and, in some instances, hyperuricemia.

PIAP-1 rising associates to a higher susceptibility to develop intrarterial thrombosis in both insulin-resistant individuals and those having developed diabetes.

As mentioned above, insulin-resistance associates to a lesser or greater degree with a wide range of metabolic disorders which involve, in the long term, health serious risks. It is clear that there is a strong connection between presence of obesity and development of insulin-action resistance, and this is what determines the susceptibility of obese people to develop frank diabetes; moreover, obesity seems to be the most common cause of insulin-resistance. It has been documented a defined connection between android-type obesity (or central, i.e., that wherein the adipose tissue accumulation is more prominent in abdomen) and a low responsiveness to insulin.

There is a great variety of medicaments used as hypoglycemic agents; the combination of drugs is usual in order to reduce glucose levels. However, in many cases, these combinations do not achieve the set goal, so the use of formulations with synergistic effect is of great usefulness in type-2 Diabetes of difficult control.

It is of interest to revise the combination of a thiazolidinedione (glitazone) such as Pioglitazone and a biguanide such as Metformin, as the combination produces synergy with considerable reductions of glucose levels in type-2 diabetes mellitus of difficult control.

### PIOGLITAZONE

Pioglitazone is an antidiabetic agent from the group of thiazolidinediones, which depends on the presence of insulin for its mechanism of action. Pioglitazone decreases insulin-resistance in periphery and liver, resulting in increased insulin-dependent glucose disposal as well as decreased hepatic glucose waste. Unlike sulfonylureas, pioglitazone is not an insulin secretagogue.

Pioglitazone is a potent agonist and highly selective for peroxisome proliferator-activated receptor gamma (PPARg). PPARg receptors are found in tissues important for insulin action, such as adipose tissue, skeletal muscle and liver. Activation of PPARg nuclear receptors modulates the transcription of a number of insulin-responsive genes involved in control of glucose and lipid metabolism.

In animal models of diabetes, pioglitazone reduces the hyperglycemia, hyperinsulinemia and hypertriglyceridemia, characteristic of insulin-resistance states such as type-2 diabetes. The metabolic changes produced by pioglitazone result in increased responsiveness of insulin-dependent tissues and are observed in numerous animal models of insulin-resistance.

Since pioglitazone increases the effects of circulating insulin (by decreasing insulin-resistance), it does not reduce blood glucose in animal models that lack endogenous insulin.

Serum concentrations of total pioglitazone (pioglitazone plus active metabolites) remain elevated during 24 hours after a single daily dose. Steady-state serum concentrations of both pioglitazone and total pioglitazone are reached within 7 days. At steady state, two of the pharmacologically active metabolites of pioglitazone, metabolites III (M-III) and IV (M-IV), reach serum concentrations equal to or greater than pioglitazone.

In both healthy volunteers and in patients with type-2 diabetes, pioglitazone comprises approximately 30% to 50% of the maximum total pioglitazone serum concentrations and from 20% to 25% of the total area under the curve of time/serum concentration (AUC). For pioglitazone and total pioglitazone, maximum serum concentration (Cmax), AUC and minimum serum concentration (Cmin) proportionately increase at doses of 15 mg and 30 mg per day. There is a slightly less than proportional increase for pioglitazone and total pioglitazone at a dose of 60 mg per day.

After oral administration at fasting, pioglitazone in serum can be measured within the first 30 minutes and maximum concentrations are observed within 2 hours. Foods slightly delays maximum serum concentration to 3 or 4 hours, but does not alter the extent of absorption.

The mean apparent distribution volume (Vd/F) of pioglitazone after the administration of a single dose is 0.63 ± 0.41 (mean ± DS) l/kg of body weight. Pioglitazone is significantly bound to serum proteins in the human (> 99%), particularly to serum albumin. Pioglitazone also binds to other serum proteins, but with lower affinity. Metabolites M-III and M-IV are also extensively bound to serum albumin (> 98%).

Pioglitazone is widely metabolized by hydroxylation and oxidation; the metabolites are partly converted to sulfate or glucoronide conjugates. Metabolites M-II and M-IV (hydroxy derivatives of pioglitazone) and M-III (keto derivative of pioglitazone) are pharmacologically active in animal models of type 2 diabetes. In addition to pioglitazone, M-III and M-IV are the principal drug-related species found in human serum after multiple doses. At steady-state, in both healthy volunteers and in patients with type 2 diabetes, pioglitazone comprises approximately 30% to 50% of the total maximum serum concentrations and 20% to 25% of the total area under the curve (AUC).

Pioglitazone incubated with human P450 or human liver microsomes results in the formation of M-IV and to a much lesser degree, M-II. The major cytochrome P-450 isoforms involved in the hepatic metabolism of pioglitazone are CYP2C8 and CYP3A4 with contributions from a variety of other isoforms including the mainly extra hepatic CYP1A1. Ketoconazole inhibits up to 85% of hepatic pioglitazone metabolism *in vitro* at a molar concentration equal to pioglitazone.

Pioglitazone did not inhibit P-450 activity when incubated with human P-450 liver microsomes. *In vivo* human studies have not been performed to investigate any induction of CYP3A4 by pioglitazone.

After oral administration, about 15% to 30% of the pioglitazone dose is recovered in the urine. Renal elimination of pioglitazone is negligible, and the drug is excreted mainly as metabolites and their conjugates. It is thought that most of the oral dose is excreted in the bile either unchanged or as metabolites, and eliminated in the feces.

The mean serum half-live of pioglitazone and total pioglitazone ranges from 3 to 7 hours and 16 to 24 hours, respectively; pioglitazone has an apparent clearance, CL/F, calculated to be 5-7 l/h.

### METFORMIN

Metformin is a biguanide provided with antihyperglycemic effects.

It reduces basal plasma and postprandial glucose levels. It does not stimulate insulin secretion and, therefore, it produces neither hypoglycemia nor hyperinsulinemia.

Metformin acts through three mechanisms:
1. It reduces glucose hepatic production by inhibiting glyconeogenesis and glycogenolysis.
2. In the muscle, it increases insulin responsiveness, improving glucose retention and utilization.
3. It delays glucose intestinal absorption.

Metformin stimulates intracellular synthesis of glycogen, acting on glycogen-synthetase. Metformin increases transport capacity of all types of membranous glucose transporters (GLUT), particularly GLUT4.

In addition to its action on glycemia, metformin produces favorable effects on lipid metabolism. This effect has been observed at therapeutic doses in short and long term clinical studies, in which metformin reduces total cholesterol, LDL cholesterol and triglyceride levels.

After its oral administration, metformin absorption is completed. The bioavailability thereof is about 50-60% in healthy individuals and the non-absorbed fraction is recovered in the feces from 20 to 30%. It shows a Tmax of 2.5 hours. At the usual dosing schedules, steady-state plasma concentrations are reached within 24 to 48 hours and are generally less than 1 µg/ml. The maximum plasma concentration is of 1.5 µg/ml and is reached within one to three hours after its administration. In controlled clinical studies, the maximum plasma concentration of metformin does not exceed 4 µg/ml, even at maximum doses. In cases of lactacidosis caused by metformin, plasma concentrations generally exceed 5 µg/ml.

Food slightly delays the absorption of metformin. After the administration of a dose of 850 mg, the Cmax is decreased at 40% and the AUC (area under the curved) at 25% and the Tmax is increased within 35 minutes. The clinical relevance of these data is unknown.

Plasma protein binding is negligible. Maximum concentration in blood is lower than that in plasma and appears at approximately at the same time. Red blood cells seem to represent a secondary compartment of distribution. The mean volume distribution is between 63 to 276 1.

Metformin is excreted in the urine without changes and does not suffer neither hepatic metabolism (metabolites in humans have not been identified) nor bile excretion.

Metformin renal clearance is > 400 ml/min, i.e., about 3.5 times higher than creatinine clearance, which indicates that metformin is eliminated by glomerular filtration and tubular secretion. After an oral administration, about 90% of the absorbed drug is eliminated in the urine within 24 hours, with a plasma elimination half-life of about 6.2 hours. In blood, the elimination half-life is about 17.6 hours, which supports that erythrocyte mass can be a distribution compartment.

In patients with renal function deterioration (based on creatinine clearance) the plasma half-life of metformin is extended and renal clearance is proportionately decreased to creatinine clearance.

Metformin is dialyzable, with a clearance up to 170 ml/min under a suitable hemodialysis. Therefore, this approach may be useful for removal of the accumulated drug when an overdosage is suspected.

When obesity and diabetes combine, which is quite frequent, the treatment of these patients represents authentic clinical challenge.

More than 80% of the patients with type-2 diabetes mellitus present overweight. Overweight has a negative effect over glycemic control, lipid disorders, arterial hypertension and other risk factors highly prevalent in patients with DM2; it is quite frequent that multiple drugs are prescribed for glycemic control. However, in many cases they make other risk factors to become worse and this deteriorates even more the patient's physical shape. With this object, trials were conducted with the combination of Pioglitazone/Metformin, with the aim to assess the effectiveness in glycemic rates as well as the tolerance thereof by assessing the adverse effects, using a combination producing a synergistic effect in glucose basal levels.

### OBJECTS OF THE INVENTION

One of the objectives of the present invention is to achieve a pharmaceutical composition for treating type-2 Diabetes mellitus which permits a fewer amount of the components than when they are separately administered, so fewer side effects are prevented.

Another objective is to achieve the above by reaching the same performance or an even better performance with these fewer amounts.

Other objects and advantages of the present invention would be apparent from the study of the following description and accompanying drawings with illustrative purposes only rather than limitative.

### BRIEF DESCRIPTION OF THE INVENTION

Generally, the materialization of the invention is found in a pharmaceutical combination comprising a combination of derivate substances of thiazolidinediones (glitazones) such as Pioglitazone and a biguanide such as Metformin, for pharmaceutical use of the composition in type-2 Diabetes Mellitus; the combination produces a combined product with synergistic properties. In one of the embodiments, pharmaceutically acceptable salts thereof substitute the active substances.

In different trials conducted for defining the present invention it could be determined that the combination of the ingredients offers a product with improved properties, which requires a fewer amount of at least one ingredient and produces a synergistic effect, being able to significantly improve the glucose indexes, cholesterol and triglyceride indexes without adverse effects.

In one of the embodiments of the present invention, metformin is present as metformin hydrochloride while pioglitazone is present in a range from 15 to 45 mg per dosage unit.

Another embodiment of the present invention is materialized in a composition in which pioglitazone is present in a concentration of 30 mg per dosage unit.

As for metformin, one preferred embodiment is materialized in a pharmaceutical composition in which metformin hydrochloride is present in a concentration of 500 mg per dosage unit.

The last trials were not experimented with substances such as: Rosiglitazone, Nateglinide, Repaglinide, Glibenclamide, Glicazide, Glimepiride, and Acarbose.

### DETAILED DESCRIPTION OF THE INVENTION

In accordance with the present invention, the pharmaceutical composition comprising a combination of a thiazolidinedione (glitazone) known as Pioglitazone and a biguanide such as Metformin was made, whose combination produces a synergistic effect in glucose index reduction, being better tolerated, and with fewer adverse effects in patients with type-2 Diabetes Mellitus.

### EXAMPLE 1

A multicentric, randomized, double blind placebo-controlled trial was conducted. In which 600 individuals diagnosed with DM2 and obesity with a BMI higher than 35 kg/m² were included. The patients were randomized to three treatment groups; group 1 received the combination Pioglitazone/Metformin during 8 weeks, group 2 received pioglitazone plus placebo during 8 weeks and group 3 received metformin plus placebo during 8 weeks.

### RESULTS

The combination pioglitazone/metformin significantly reduced HbA_{1c} and fasting glycemia, compared to pioglitazone-placebo and metformin/placebo, a decrease in triglycerides and an increase in HDL cholesterol was also observed, which were also significant compared to pioglitazone/placebo and metformin/placebo.

The patients' weight reduced in the group of pioglitazone/metformin (-1.36kg) and increased in the group of pioglitazone/placebo and metformin/placebo.

In a 2% adverse effects were observed characterized by nausea and vomit.

### CONCLUSIONS

The reduction in basal glucose indexes in individuals with DM2 presenting obesity has been tested in this clinical trial by achieving the reduction of concomitant risk factors in this type of patients, such as reduction of triglycerides and increase of HDL cholesterol, which provides cardiac reliability and significant weight loss showed in the group of individuals who received the combination Pioglitazone/Metformin, establishing an excellent effectiveness and excellent tolerance thereof with the minimum adverse events reported.

### EXAMPLE 2

A multicentric, randomized, double blind placebo-controlled trial was conducted. In which 300 patients diagnosed with DM2 and obesity were included. The object of the study was to establish the reduction in lipid profile and in overweight. Three treatment groups were randomized; group 1 received the combination Pioglitazone/Metformin, group 2 received the combination pioglitazone/placebo, and group 3 received metformin/placebo during 12 weeks.

### RESULTS

The reduction in triglycerides and the increase of HDL cholesterol was statistically significant for group 1 compared to the reported levels in the admission, compared to group 2 and 3, wherein no significant reduction was reported.

The weight loss was evident in the group who received the combination pioglitazone/metformin with a reduction of 2.5 kg compared to pioglitazone/placebo, metformin/placebo groups.

### CONCLUSIONS

The reduction of co-morbid factors involved in the pathogenesis of patients with diabetes mellitus with the combination pioglitazone/metformin was evident, considerably reducing vascular risk factors and DM2 complications, with minimum adverse effects.

## Claims

1. Synergistic pharmaceutical composition comprising a synergistic combination of a thiazolidinedione (glitazone) and a biguanide for treating type-2 Diabetes Mellitus.

2. Pharmaceutical composition according to claim 1 comprising the combination of a thiazolidinedione (glitazone) known as Pioglitazone and/or any of the salts thereof, and a biguanide known as metformin and/or any salts thereof.

3. A pharmaceutical composition according to claim 2 wherein the ingredient combination provides a product with improved properties, which requires a fewer amount of at least one ingredient and produces a synergistic effect.

4. A pharmaceutical composition according to claim 2 whose ingredient combination significantly improves glucose indexes, cholesterol and triglycerides indexes, without adverse events.

5. A pharmaceutical composition according to claims 2, 3, and 4, wherein in the ingredient requiring a fewer dose of acid is Metformin.

6. A pharmaceutical composition according to claim 5, wherein metformin is in the form of metformin hydrochloride.

7. A pharmaceutical composition according to claim 2, wherein pioglitazone is present in a range from 15 to 45 mg per dosage unit.

8. A pharmaceutical composition according to claim 7, wherein pioglitazone is present in a concentration of 30 mg per dosage unit.

9. A pharmaceutical composition according to claim 2, wherein metformin hydrochloride is present in a concentration of 500 mg per dosage unit.

10. Pharmaceutical compositions according to all previous claims, wherein substances such as: Rosiglitazone, Nateglinide, Repaglinide, Glibenclamide, Glicazide, Glimepiride, Acarbose are excluded from the formulation.

11. The use of the compositions according to claim 2 for treating type-2 diabetes mellitus.
